(19) Europäisches Patentamt
European Patent Office
Office européen des brevets

(11) **EP 4 000 539 A1**

(12) **EUROPEAN PATENT APPLICATION**
published in accordance with Art. 153(4) EPC

(43) Date of publication:
**25.05.2022 Bulletin 2022/21**

(21) Application number: **20841266.8**

(22) Date of filing: **06.07.2020**

(51) International Patent Classification (IPC):
***A61B 17/12*** $^{(2006.01)}$

(52) Cooperative Patent Classification (CPC):
**A61B 17/12**

(86) International application number:
**PCT/JP2020/026491**

(87) International publication number:
**WO 2021/010222 (21.01.2021 Gazette 2021/03)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.07.2019 JP 2019132985**

(71) Applicant: **ASAHI INTECC CO., LTD.
Seto-shi, Aichi 489-0071 (JP)**

(72) Inventor: **THASATAN Witcha
Seto-shi, Aichi 489-0071 (JP)**

(74) Representative: **Winter, Brandl - Partnerschaft
mbB
Alois-Steinecker-Straße 22
85354 Freising (DE)**

(54) **IMPLANT DEVICE**

(57) Provided is an implant device with which an implant portion can be stably placed within a blood vessel. The implant device 1 comprises: a connection coil body 21 having an inner cavity with a first inner diameter r1; an implant coil body 22 arranged more toward a distal end direction of the implant device 1 than a distal end portion of the connection coil body 21, and having a second inner diameter r2; a first wire 14 having a small diameter portion 14A having a first outer diameter R1, and a large diameter portion 14B having a second outer diameter R2 that is larger than the first outer diameter R1; and a second wire 15 having a third outer diameter R3.

FIG. 3

**EP 4 000 539 A1**

## Description

TECHNICAL FIELD

The present disclosure relates to an implant device.

BACKGROUND ART

Currently, embolization is used as a treatment method for aneurysms and the like, and is performed by placing a coil or the like made of a metal that does not adversely affect the body inside a blood vessel. As an instrument used in the embolization, for example, an instrument provided with an implant and a positioning device having a positioner and an implant interface has been proposed (for example, see Patent Literature 1).

The instrument disclosed in Patent Literature 1 is configured such that, by pulling in a cord that is blocking a portion of the port of the implant interface toward the near side, the ball of a rod which is connected to a thread of the implant is released from the port, causing the implant to become detached from the positioning device.

CITATION LIST

Patent Literature

Patent Literature 1: Japanese Unexamined Patent Application Publication (Translation of PCT Application) No. 2010-521331

SUMMARY OF INVENTION

TECHNICAL PROBLEM

However, in the instrument described above, when the ball of the rod of the implant is released from the port of the implant interface, a restoring force occurs in the thread that connects the holder of the implant and the rod, which may cause the position of the implant to be unstable.

An object of the present disclosure is to provide an implant device with which an implant portion can be stably placed within a blood vessel.

SOLUTION TO PROBLEM

In order to solve the above object, an implant device, which is an aspect of the present disclosure, includes: a first coil body having an inner cavity with a first inner diameter r1 that passes through from a proximal end to a distal end; a second coil body arranged more toward a distal end direction of the implant device than a distal end portion of the first coil body, and having a second inner diameter r2; a first wire having a small diameter portion having a first outer diameter R1, and a large diameter portion connected to the small diameter portion, and having a second outer diameter R2 that is larger than the first outer diameter R1; and a second wire having a third outer diameter R3; wherein the small diameter portion of the first wire and the second wire can be simultaneously inserted into the inner cavity of the first coil body, and the first outer diameter R1, the second outer diameter R2, the third outer diameter R3, and the first inner diameter r1 satisfy equation (1): $(R1+R2)/2+R3 > r1 > R1+R3$, and equation (2): $r1 > R2$.

The implant device further includes a third wire that is located on the proximal end side of the second wire, connected to the second wire, and capable of moving a predetermined distance along a long axis direction of the second wire.

The second outer diameter R2, the third outer diameter R3, and the second inner diameter r2 may satisfy equation (3): $r2 > (R1+R2)/2+R3$.

ADVANTAGEOUS EFFECTS OF INVENTION

According to the present disclosure, it is possible to provide an implant device with which an implant portion can be stably placed within a blood vessel.

BRIEF DESCRIPTION OF DRAWINGS

FIG. 1 is an overall view of an implant device according to an embodiment.
FIG. 2 is a cross-sectional view of a part of the implant device.
FIG. 3(a) is an explanatory diagram of the dimensions of a first wire, a second wire, a connection coil body, and an implant coil body, and FIG. 3(b) is an explanatory diagram of the relationship between the dimensions of the first wire, the second wire, and the connection coil body.
FIG. 4 is an explanatory diagram of a method of separating an implant portion from a delivery wire.

EMBODIMENTS OF THE INVENTION

Hereinafter, an implant device according to an embodiment of the present disclosure will be described with reference to the drawings, but the present invention is not limited only to the embodiments illustrated in the drawings. In the present disclosure, a distal end refers to an end portion of the implant device in which a distal tip is located, and a proximal end refers to an end portion on the opposite side to the distal end.

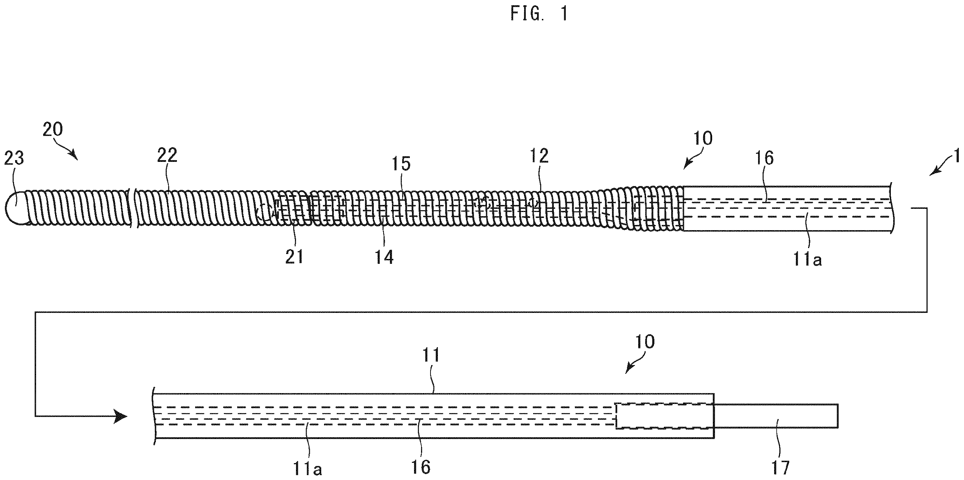

FIG. 1 is an overall view of an implant device 1 according to the present embodiment. Note that, in FIG. 1, illustration of the coating layer 13 has been omitted.

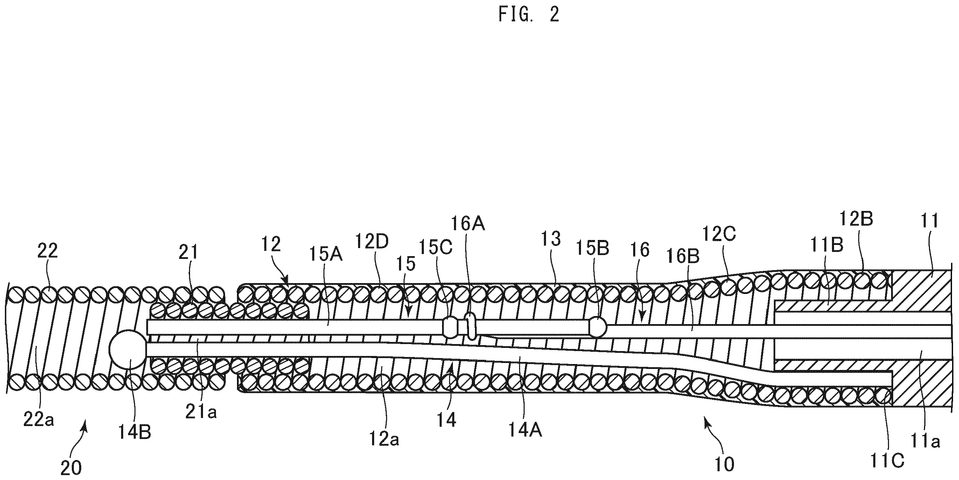

FIG. 2 is a cross-sectional view of a part of the implant device 1.

As shown in FIG. 1, the implant device 1 includes a delivery wire 10 and an implant portion 20.

<Delivery Wire 10>

The delivery wire 10 includes a shaft 11, a delivery coil body 12, a coating layer 13, a first wire 14, a second wire 15, a third wire 16, and an operation shaft 17.

The shaft 11 is a long, linear (wire-like) member having flexibility, and has a hollow shape in which an inner cavity 11a is formed that passes through from the proximal end to the distal end. A reduced diameter portion 11B whose outer diameter is reduced is provided in the distal end portion of the shaft 11. The proximal end portion of the shaft 11 functions as a grip portion which is gripped by an operator.

The material forming the shaft 11 is not particularly limited as long it is a material having a level of flexibility that enables it to easily follow the curvature of the lumen inside the body (such as a blood vessel), and stainless steel, superelastic alloy materials such as nickel-titanium alloy, and synthetic resins such as polyvinyl chloride resins, urethane resins, polyolefin resins, polyamide resins, and fluororesins can be adopted as the material. The dimension of the shaft 11 is appropriately set according to the treatment target site; for example, when used for treatment of a cerebral aneurysm, the total length is set to about 100 to 2000 mm, and the outer diameter is set to about 0.2 to 2 mm.

The delivery coil body 12 is provided on the distal end side of the shaft 11. The delivery coil body 12 is formed by spirally winding one or more wires so that adjacent wires make close contact with each other, and has a hollow shape having an inner cavity 12a that passes through from the proximal end to the distal end. The proximal end of the delivery coil body 12 is joined to a stepped surface 11C of the shaft 11. The reduced diameter portion 11B of the shaft 11 is inserted into a proximal end portion 12B of the delivery coil body 12. The delivery coil body 12 has a tapered portion 12C on the distal end side of the proximal end portion 12B, and a distal end portion 12D having a substantially constant outer diameter on the distal end side of the tapered portion 12C.

The material forming the delivery coil body 12 is not particularly limited as long it is a material having a level of flexibility that enables it to easily follow the curvature of the lumen inside the body (such as a blood vessel), and stainless steel, superelastic alloy materials such as nickel-titanium alloy, and radiopaque metals such as platinum and tungsten can be adopted as the material. The dimensions of the delivery coil body 12 are appropriately set according to the treatment target site; for example, when used for treatment of a cerebral aneurysm, the total length is set to 0.5 to 200 mm, and the outer diameter is set to about 0.3 to 2 mm. Furthermore, the diameter of the wires constituting the delivery coil body 12 is about 0.01 to 0.1 mm.

The coating layer 13 covers the entire outer peripheral surface of the delivery coil body 12. The coating layer 13 is, for example, a heat shrink tube, and examples of the resin constituting the coating layer 13 include biocompatible resin materials such as polyamide resins and fluororesins, and hydrophilic coating materials.

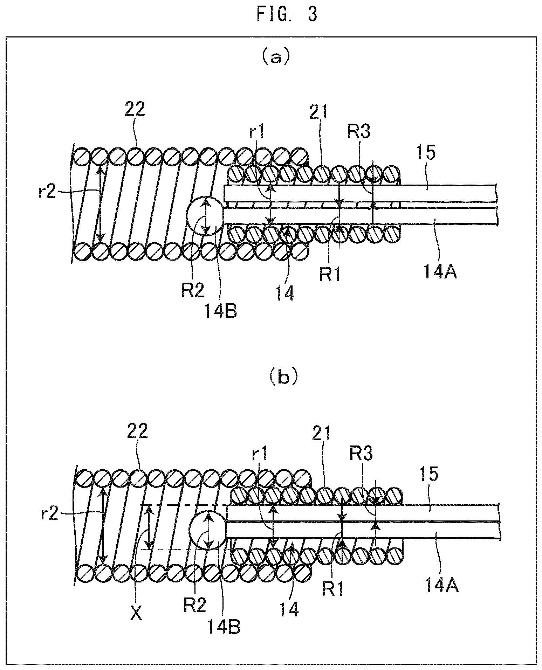

The first wire 14 has a small diameter portion 14A and a large diameter portion 14B. The small diameter portion 14A has a substantially constant first outer diameter R1 (FIG. 3) from the proximal end to the distal end. The proximal end of the small diameter portion 14A is located on the inside of the delivery coil body 12 and joined to the stepped surface 11C of the shaft 11, and the small diameter portion 14A extends from the stepped surface 11C of the shaft 11 through the inner cavity 12a of the delivery coil body 12 toward the distal end. The proximal end portion of the small diameter portion 14A is joined to the outer peripheral surface of the reduced diameter portion 11B of the shaft 11 and the inner peripheral surface of the delivery coil body 12. As a result, the first wire 14, the shaft 11, and the delivery coil body 12 are integrated. The distal end portion of the small diameter portion 14A protrudes from the inner cavity 12a of the delivery coil body 12. That is to say, the small diameter portion 14A is configured to be longer than the delivery coil body 12.

The large diameter portion 14B is connected to the distal end of the small diameter portion 14A, and has a second outer diameter R2 (FIG. 3) that is larger than the first outer diameter R1. The large diameter portion 14B is located on the outside of the delivery coil body 12. As shown in FIG. 2, when the delivery wire 10 and the implant portion 20 are connected, the distal end portion of the first wire 14 passes through a connection coil body 21 described below, and the large diameter portion 14B is located inside an inner cavity 22a of an implant coil body 22 described below. The total length of the first wire 14 is, for example, about 0.5 to 200 mm, the outer diameter of the small diameter portion 14A is, for example, about 0.1 to 0.5 mm, and the outer diameter of the large diameter portion 14B is, for example, about 0.1 to 1 mm.

The shape of the large diameter portion 14B is a ball shape in the present embodiment, but may be any other shape. The outer diameter of the large diameter portion 14B is the maximum length in a direction perpendicular to an axial direction of the first wire 14 in a cross-section taken in a direction perpendicular to the axial direction of the first wire 14. According to this definition of the outer diameter, it is possible to identify the maximum length and determine the outer diameter of the large diameter portion 14B, even when the large diameter portion 14B is, for example, disc-shaped, a long spherical shape, or cone-shaped.

The second wire 15 includes a wire portion 15A, a proximal end side bulging portion 15B, and a distal end side bulging portion 15C. The wire portion 15A has a substantially constant third outer diameter R3 (FIG. 3) from the proximal end to the distal end. The proximal end side bulging portion 15B is provided on the proximal end of the wire portion 15A, and has an outer diameter which is larger than the third outer diameter R3. The distal end side bulging portion 15C is provided at a position of the wire portion 15A which is a predetermined distance on the distal end side from the proximal end side bulging portion 15B. The distal end side bulging portion 15C has an outer diameter which is larger than the third outer diameter R3.

As shown in FIG. 2, when the delivery wire 10 and the implant portion 20 are connected, the distal end portion of the second wire 15 is inserted inside the connection coil body 21 described below. The total length of the second wire 15 is for example, about 0.5 to 200 mm, and the outer diameter is, for example, about 0.1 to 1 mm.

The third wire 16 includes a connection portion 16A and a wire portion 16B. The connection portion 16A has a substantially annular shape, and is attached to the outer periphery of the wire portion 15A between the proximal end side bulging portion 15B and the distal end side bulging portion 15C of the second wire 15. The inner diameter of the connection portion 16A is larger than the outer diameter of the wire portion 15A of the second wire 15, and smaller than the outer diameter of the proximal end side bulging portion 15B and the distal end side bulging portion 15C. As a result, the connection portion 16A is configured to be capable of sliding a predetermined distance along the long axis direction of the wire portion 15A of the second wire 15 without being released from the wire portion 15A of the second wire 15. The predetermined distance is, for example, about 0.5 to 200 mm.

The wire portion 16B has a substantially constant outer diameter from the proximal end to the distal end, has the proximal end connected to the operation shaft 17 described below, extends from the operation shaft 17 toward the earlier application side inside the inner cavity 11a of the shaft 11 and the inner cavity 12a of the delivery coil body 12, and the distal end of the wire portion 16B is connected to the connection portion 16A. The third wire 16 has the connection portion 16A attached to the outer periphery of the wire portion 15A of the second wire 15, and therefore, the third wire 16 is capable of moving a predetermined distance relative to the second wire 15 along the long axis direction of the second wire 15. The total length of the third wire 16 is for example, about 100 to 200 mm, and the outer diameter is, for example, about 0.1 to 1.0 mm.

As examples of the material forming the first wire 14, the second wire 15, and the third wire 16, stainless steel, superelastic alloy materials such as nickel-titanium alloy, and radiopaque metals such as platinum and tungsten can be adopted.

The operation shaft 17 has a substantially cylindrical shape, and the distal end portion of the operation shaft 17 is inserted into the shaft 11 from the proximal end side of the inner cavity 11a of the shaft 11. The operation shaft 17 is slidable and movable with respect to the shaft 11. The third wire 16 is connected to the operation shaft 17. Therefore, by moving the operation shaft 17 with respect to the shaft 11 in the long axis direction, the third wire 16 also moves along the long axis direction. As examples of the material constituting the operation shaft 17, the same materials as those constituting the shaft 11 may be adopted.

<Implant Portion 20>

The implant portion 20 includes a connection coil body 21, an implant coil body 22, and a distal end portion 23.

The connection coil body 21 is formed by spirally winding one or more wires so that adjacent wires make close contact with each other, and has a hollow shape having an inner cavity 21a with a first inner diameter r1 (FIG. 3) that passes through from the proximal end to the distal end. As shown in FIG. 2, when the delivery wire 10 and the implant portion 20 are connected, the proximal end portion of the connection coil body 21 is inserted into the inner cavity 12a of the delivery coil body 12. Note that the proximal end portion of the connection coil body 21 is not joined to the delivery coil body 12, and the connection coil body 21 is separable from the delivery coil body 12. The connection coil body 21 corresponds to the first coil body.

The dimensions of the connection coil body 21 are appropriately set according to the treatment target site; for example, when used for treatment of a cerebral aneurysm, the total length is set to about 0.5 to 100 mm, and the inner diameter (first inner diameter r1) is set to about 0.2 to 1 mm. Furthermore, the diameter of the wires constituting the connection coil body 21 is about 0.01 to 0.1 mm.

The implant coil body 22 is formed by spirally winding one or more wires so that adjacent wires make close contact with each other, and has a hollow shape having an inner cavity 22a with a second inner diameter r2 (FIG. 3) that passes through from the proximal end to the distal end. The implant coil body 22 is disposed from the distal end portion of the connection coil body 21 toward the distal end direction. The distal end portion of the connection coil body 21 is inserted and joined to the proximal end portion of the implant coil body 22. The implant coil body 22 corresponds to the second coil body.

The dimensions of the implant coil body 22 are appropriately set according to the treatment target site; for example, when used for treatment of a cerebral aneurysm, the total length is set to about 10 to 500 mm, and the inner diameter (second inner diameter r2) is set to about 0.4 to 2 mm. Furthermore, the diameter of the wires constituting the implant coil body 22 is about 0.01 to 0.1 mm.

The distal end portion 23 is substantially ball-shaped, and is joined to the distal end of the implant coil body 22 so as to block the opening of the distal end of the implant coil body 22. The outer diameter of the distal end portion 23 is the same or slightly smaller than the outer diameter of the implant coil body 22. As examples of the material forming the connection coil body 21, the implant coil body 22, and the distal end portion 23, radiopaque metals such as platinum, gold, tungsten, and alloys of these metals can be adopted.

The wires constituting the delivery coil body 12, the connection coil body 21, and the implant coil body 22 may be a single wire or a twisted wire in which a plurality of single wires are twisted together.

Next, the relationship between the dimensions of the first wire 14, the second wire 15, the connection coil body 21, and the implant coil body 22 will be described.

FIG. 3(a) is an explanatory diagram of the dimensions of the first wire 14, the second wire 15, the connection coil body 21, and the implant coil body 22, and FIG. 3(b) is an explanatory diagram of the relationship between the dimensions of the first wire 14, the second wire 15, and the connection coil body 21.

As shown in FIG. 3(a), the small diameter portion 14A of the first wire 14 has the first outer diameter $R1$, the large diameter portion 14B of the first wire 14 has the second outer diameter $R2$, the second wire 15 has the third outer diameter $R3$, the connection coil body 21 has the first inner diameter $r1$, and the implant coil body 22 has the second inner diameter $r2$.

Further, the first wire 14, the second wire 15, and the connection coil body 21 are configured such that the first outer diameter $R1$ of the small diameter portion 14A, the second outer diameter $R2$ of the large diameter portion 14B, the third outer diameter $R3$ of the second wire 15, and the first inner diameter $r1$ of the connection coil body 21 satisfy equations (1) and (2) below.

$$(R1+R2)/2+R3 > r1 > R1+R3 \quad \cdots (1)$$

$$r1 > R2 \quad \cdots (2)$$

The term $[(R1+R2)/2+R3]$ in equation (1) corresponds to a maximum width X of the first wire 14 and the second wire 15 when the first wire 14 and the second wire 15 are viewed from the distal end side in a state where the small diameter portion 14A of the first wire 14 and the second wire 15 are in contact with each other as shown in FIG. 3(b), Because the relationship $(R1+R2)/2+R3 > r1$ shown in equation (1) is satisfied, the first wire 14 and the second wire 15 are configured so as to not become released from the connection coil body 21, even in a state where the small diameter portion 14A of the first wire 14 and the second wire 15 are in contact with each other. Because the relationship $r1 > R1+R3$ shown in equation (1) is satisfied, both the small diameter portion 14A of the first wire 14 and the second wire 15 can be simultaneously inserted into the connection coil body 21.

Further, because the relationship $r1 > R2$ shown in equation (2) is satisfied, the first wire 14 can be released from the connection coil body 21 after the second wire 15 is released from the connection coil body 21.

Furthermore, the first wire 14, the second wire 15, and the implant coil body 22 are configured such that the second outer diameter $R2$ of the large diameter portion 14B, the third outer diameter $R3$ of the second wire 15, and the second inner diameter $r2$ of the implant coil body 22 satisfy equation (3) below.

$$r2 > (R1+R2)/2+R3 \quad \cdots (3)$$

Because the second inner diameter $r2$, the first outer diameter $R1$, the second outer diameter $R2$, and the third outer diameter $R3$ satisfy the relationship shown in equation (3), the small diameter portion 14A of the first wire 14 and the second wire 15 can be simultaneously inserted into the connection coil body 21 inside the implant coil body 22.

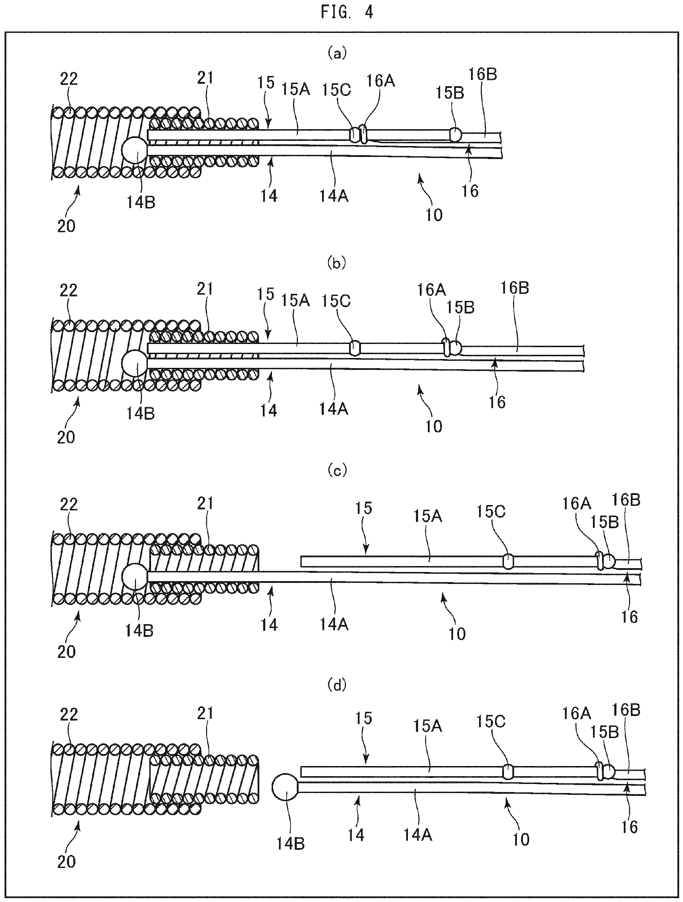

Next, a method of separating the implant portion 20 from the delivery wire 10 in the implant device 1 of the present embodiment will be described with reference to FIG. 4.

FIG. 4 is an explanatory diagram of a method of separating the implant portion 20 from the delivery wire 10. In FIG. 4, the illustration of the delivery coil body 12 and the coating layer 13 is omitted.

The operation shaft 17 shown in FIG. 1 is positioned in the shaft 11 at the most distal end of the range of possible movement, and as shown in FIG. 4(a), the distal end side bulging portion 15C of the second wire 15 is pushed by the connection portion 16A of the third wire 16 so as to advance the implant device 1 inside the blood vessel in a state where the wire portion 15A of the second wire 15 is located inside the connection coil body 21, which causes the implant coil body 22 to be inserted into the target site (such as a cerebral aneurysm).

Then, the operation shaft 17 is pulled so as to move the third wire 16 by a predetermined distance toward the proximal end side with respect to the second wire 15, and as shown in FIG. 4(b), the connection portion 16A of the third wire 16 is brought into contact with the proximal end side bulging portion 15B of the second wire 15. Further, as shown in FIG. 4(c), by pulling the operation shaft 17, the second wire 15 is also pulled via the third wire 16, and the second wire 15 becomes released from the connection coil body 21.

Finally, by pulling the shaft 11 toward the proximal end side, the first wire 14 is also pulled such that, as shown in FIG. 4(d), the first wire 14 is released from the connection coil body 21. As a result, the implant portion 20 is separated from the delivery wire 10, and the implant portion 20 is placed in the target site.

As described above, according to the implant device 1 of the present disclosure, the first outer diameter $R1$ of the small diameter portion 14A, the second outer diameter R2 of the large diameter portion 14B, the third outer diameter R3 of the second wire 15, and the first inner diameter r1 of the connection coil body 21 are configured so as to satisfy the relationships of equation (1) and equation (2) above. With this configuration, the small diameter portion 14A of the first wire 14 and the second wire 15 can be simultaneously inserted into the connection coil body 21, and the first wire 14 and the second wire 15 can be prevented from being released from the connection coil body 21, even in a state where the small diameter portion 14A of the first wire 14 and the second wire 15 are in contact with each other. Further, the first wire 14 can be released from the connection coil body 21 after the second wire 15 is released from the connection coil body 21. As a result, it is possible to suppress force from acting on the implant portion 20 when the implant portion 20 (the connection coil body 21 and the implant coil body 22) is separated from the delivery wire 10 (the first wire 14, the second wire 15, and the third wire 16), and therefore, the implant portion 20 can be stably placed within a blood vessel to perform embolization.

The implant device 1 includes the third wire 16, which is located on the proximal end side of the second wire 15, is connected to the second wire 15, and is capable of moving a predetermined distance relative to the second wire 15 along the long axis direction of the second wire 15. Therefore, when the implant device 1 is inserted inside a blood vessel, the second wire 15 can be prevented from being released from the connection coil body 21, even when the third wire 16 moves toward the proximal end side with respect to the second wire 15.

Furthermore, because the second outer diameter R2, the third outer diameter R3, and the second inner diameter r2 are configured so as to satisfy equation (3), the small diameter portion 14A of the first wire 14 and the second wire 15 can be simultaneously inserted into the connection coil body 21 inside the implant coil body 22.

Note that the present disclosure is not limited to the configurations of the embodiments described above, but is stipulated by the claims, and the present disclosure is intended to include all modifications within the meaning and scope equivalent to the claims.

DESCRIPTION OF REFERENCE NUMERALS

1: Implant device
10: Delivery wire
11: Shaft
11a: Inner cavity
11B: Reduced diameter portion
11C: Stepped surface
12: Delivery coil body
12a: Inner cavity
12B: Proximal end portion
12C: Tapered portion
12D: Distal end portion
13: Coating layer
14: First wire
14A: Small diameter portion
14B: Large diameter portion
15: Second wire
15A: Wire portion
15B: Proximal end side bulging portion
15C: Distal end side bulging portion
16: Third wire
16A: Connection portion
16B: Wire portion
17: Operation shaft
20: Implant portion
21: Connection coil body
21a: Inner cavity
22: Implant coil body
22a: Inner cavity
r1: First inner diameter
r2: Second inner diameter
R1: First outer diameter
R2: Second outer diameter
R3: Third outer diameter

Claims

1. An implant device, comprising:

    a first coil body having an inner cavity with a first inner diameter r1 that passes through from a proximal end to a distal end;
    a second coil body arranged more toward a distal end direction of the implant device than a distal end portion of the first coil body, and having a second inner diameter r2;
    a first wire having a small diameter portion having a first outer diameter R1, and a large diameter portion connected to the small diameter portion, and having a second outer diameter R2 that is larger than the first outer diameter R1; and
    a second wire having a third outer diameter R3; wherein
    the small diameter portion of the first wire and the second wire can be simultaneously inserted into the inner cavity of the first coil body, and the first outer diameter R1, the second outer diameter R2, the third outer diameter R3, and the first inner diameter r1 satisfy equation (1): $(R1+R2)/2+R3 > r1 > R1+R3$, and equation (2): $r1 > R2$.

2. The implant device according to claim 1, further comprising
a third wire located on the proximal end side of the second wire, which is connected to the second wire, and is capable of moving a predetermined distance along a long axis direction of the second wire.

3. The implant device according to claim 1 or 2, wherein the second outer diameter R2, the third outer diameter R3, and the second inner diameter r2 satisfy equation (3): r2 > (R1+R2)/2+R3.

FIG. 1

FIG. 2

EP 4 000 539 A1

FIG. 3

(a)

(b)

FIG. 4

(a)

(b)

(c)

(d)

## INTERNATIONAL SEARCH REPORT

| | International application No. |
|---|---|
| | PCT/JP2020/026491 |

A. CLASSIFICATION OF SUBJECT MATTER
Int. Cl. A61B17/12(2006.01)i
FI: A61B17/12

According to International Patent Classification (IPC) or to both national classification and IPC

B. FIELDS SEARCHED

Minimum documentation searched (classification system followed by classification symbols)
Int. Cl. A61B17/12

Documentation searched other than minimum documentation to the extent that such documents are included in the fields searched
Published examined utility model applications of Japan    1922–1996
Published unexamined utility model applications of Japan    1971–2020
Registered utility model specifications of Japan    1996–2020
Published registered utility model applications of Japan    1994–2020

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2010-523260 A (BOSTON SCIENTIFIC LTD.) 15 July 2010, paragraphs [0051]–[0059], fig. 1–5B | 1-3 |
| Y | US 2015/0335333 A1 (JONES, Donald K.) 26 November 2015, paragraphs [0079]–[0092], fig. 8–14 | 1-3 |
| Y | US 2017/0367709 A1 (COVIDIEN L.P.) 28 December 2017, paragraphs [0082]–[0084], fig. 6 | 3 |
| A | JP 8-252324 A (TARGET THERAPEUTICS INC.) 01 October 1996, paragraphs [0036]–[0047], fig. 1A–1B | 1-3 |
| A | JP 11-244388 A (B BRAUN CELSA SA) 14 September 1999, paragraphs [0014]–[0027], fig. 1–5 | 1-3 |

☒ Further documents are listed in the continuation of Box C.    ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 08.09.2020 | 24.09.2020 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

<table>
<tr><td colspan="2" rowspan="2"><strong>INTERNATIONAL SEARCH REPORT</strong></td><td>International application No.</td></tr>
<tr><td>PCT/JP2020/026491</td></tr>
</table>

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2016-36738 A (DEPUY SYNTHES PRODUCTS INC.) 22 March 2016, paragraphs [0024]-[0040], fig. 1-5 | 1-3 |
| A | JP 2016-526440 A (W. L. GORE & ASSOCIATES, INC.) 05 September 2016, paragraphs [0018]-[0053], fig. 1A-4E | 1-3 |
| A | US 2016/0008003 A1 (COVIDIEN L.P.) 14 January 2016, paragraphs [0056]-[0061], fig. 7, 8 | 1-3 |

Form PCT/ISA/210 (continuation of second sheet) (January 2015)

| INTERNATIONAL SEARCH REPORT | International application No. |
|---|---|
| Information on patent family members | PCT/JP2020/026491 |

| Patent Documents referred to in the Report | Publication Date | Patent Family | Publication Date |
|---|---|---|---|
| JP 2010-523260 A | 15.07.2010 | US 2010/0121350 A1<br>paragraphs [0056]-[0064], fig. 1-5B<br>WO 2008/127525 A1<br>CA 2681663 A1 | |
| US 2015/0335333 A1 | 26.11.2015 | WO 2014/107529 A2 | |
| US 2017/0367709 A1 | 28.12.2017 | WO 2018/005042 A1 | |
| JP 8-252324 A | 01.10.1996 | US 5578074 A<br>column 4, line 32 to column 6, line 47, fig. 1a-1b<br>EP 717961 A1 | |
| JP 11-244388 A | 14.09.1999 | US 6039744 A<br>column 2, line 64 to column 4, line 47, fig. 1-5<br>EP 925763 A1<br>FR 2772592 A1 | |
| JP 2016-36738 A | 22.03.2016 | US 2016/0038150 A1<br>paragraphs [0030]-[0046], fig. 1-5<br>EP 2982317 A1<br>CA 2898487 A1<br>CN 105361918 A<br>KR 10-2016-0018398 A | |
| JP 2016-526440 A | 05.09.2016 | US 2015/0005808 A1<br>paragraphs [0042]-[0077], fig. 1A-4E<br>WO 2014/210280 A2<br>CA 2916473 A1<br>CN 105392431 A<br>KR 10-2016-0024977 A | |
| US 2016/0008003 A1 | 14.01.2016 | WO 2014/145012 A2<br>CN 105142543 A | |

Form PCT/ISA/210 (patent family annex) (January 2015)

**EP 4 000 539 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010521331 W